(19) Europäisches Patentamt
European Patent Office
Office européen des brevets

(11) Veröffentlichungsnummer: **0 594 022 A1**

(12) **EUROPÄISCHE PATENTANMELDUNG**

(21) Anmeldenummer: **93116416.4**

(51) Int. Cl.$^5$: **C07D 401/10**, A61K 31/44

(22) Anmeldetag: **11.10.93**

(30) Priorität: **23.10.92 DE 4235943**
**08.06.93 DE 4319040**

(43) Veröffentlichungstag der Anmeldung:
**27.04.94 Patentblatt 94/17**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Fey, Peter, Dr.**
**Am Eickhof 23**
**D-42111 Wuppertal(DE)**
Erfinder: **Dressel, Jürgen, Dr.**
**Tuchstrasse 48**
**D-42477 Radevormwald(DE)**
Erfinder: **Hanko, Rudolf, Dr.**
**Schillerstrasse 23**
**D-40237 Düsseldorf(DE)**
Erfinder: **Hübsch, Walter, Dr.**
**Wildsteig 22**
**D-42113 Wuppertal(DE)**
Erfinder: **Krämer, Thomas, Dr.**
**Schneewittchenweg 37**
**D-42111 Wuppertal(DE)**
Erfinder: **Müller, Ulrich, E., Dr.**
**Neuer Triebel 91**
**D-42111 Wuppertal(DE)**
Erfinder: **Müller-Gliemann, Matthsias, Dr.**
**Laibacherstrasse 10**
**D-42697 Solingen-Ohligs(DE)**
Erfinder: **Beuck, Martin, Dr.**
**Trills 7**
**D-40699 Erkrath(DE)**
Erfinder: **Kazda, Stanislav, Prof. Dr.**
**Gellertweg 18**
**D-42115 Wuppertal(DE)**
Erfinder: **Wohlfeil, Stefan, Dr.**
**Tucherweg 25**
**D-40724 Hilden(DE)**
Erfinder: **Knorr, Andreas, Dr.**
**Trillser Graben 10**
**D-40699 Erkrath(DE)**
Erfinder: **Stasch, Johannes-Peter, Dr.**
**Alfred-Nobel-Strasse 109**
**D-42651 Solingen(DE)**
Erfinder: **Zaiss, Siegfried, Dr.**
**Farnweg 3**
**D-42113 Wuppertal(DE)**

(54) **Alkoxymethylsubstituierte Pyridonbiphenyle, als Angiotensin II Antagonisten.**

(57) Alkoxymethylsubstituierte Pyridonbiphenyle der allgemeinen Formel

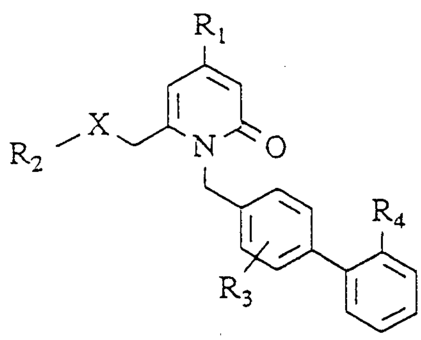

(I),

EP 0 594 022 A1

in welcher

$R^1$ für eine Carboxylgruppe steht, oder für eine $C_1$-$C_8$-Alkoxycarbonylgruppe steht,

$R^2$ für $C_1$-$C_{10}$-Alkyl (geradkettig oder verzweigt) steht, das gegebenenfalls durch Phenyl substituiert ist,

$R^3$ für Halogen, Wasserstoff, $C_1$-$C_6$-Alkyl, Hydroxy, $C_1$-$C_8$-Alkoxy, Trifluormethyl oder Trifluormethoxy steht,

$R^4$ für Carboxyl oder für Tetrazolyl steht
und

X für Sauerstoff oder Schwefel steht,
und deren Salze.

Alkoxymethylsubstituierte Pyridonbiphenyle werden hergestellt, indem man entsprechende Pyridone mit Biphenylmethylhalogenverbindungen umsetzt. Die alkoxymethylsubstituierten Pyridonbiphenyle können als Wirkstoffe in Arzneimitteln, insbesondere zur Behandlung von Bluthochdruck und Atherosklerose eingesetzt werden.

Die Erfindung betrifft alkoxymethylsubstituierte Pyridonbiphenyle, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzeilen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

In den europäischen Patentanmeldungen EP 487 745 und 500 297 werden pyridonsubstituierte Biphenyle mit blutdrucksenkenden Eigenschaften beschrieben.

Die Erfindung betrifft eine Auswahl von alkoxymethylsubstituierten Pyridonbiphenylen der allgemeinen Formel (I)

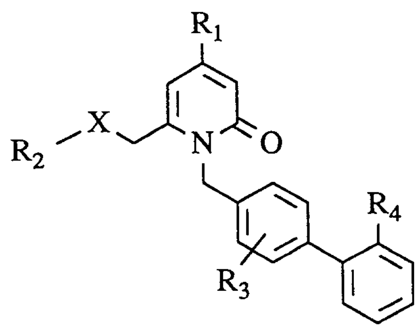

(I),

in welcher

$R^1$ für eine Carboxylgruppe steht, oder
für eine $C_1$-$C_8$-Alkoxycarbonylgruppe steht,

$R^2$ für $C_1$-$C_{10}$-Alkyl (geradkettig oder verzweigt) steht, das gegebenenfalls durch Phenyl substituiert ist,

$R^3$ für Halogen, Wasserstoff, $C_1$-$C_6$-Alkyl, Hydroxy, $C_1$-$C_8$-Alkoxy, Trifluormethyl oder Trifluormethoxy steht,

$R^4$ für Carboxyl oder
für Tetrazolyl steht,

und

X für Sauerstoff oder Schwefel steht,

und deren Salze.

Die erfindungsgemäßen alkoxymethylsubstituierten Pyridonbiphenyle können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt.

Physiologisch unbedenkliche Salze der alkoxymethylsubstituierten Pyridonbiphenyle sind im allgemeinen Metall- oder Ammoniumsalze der erfindungsgemäßen Verbindungen. Besonders bevorzugt sind z.B. Lithium-, Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomere oder als Diastereomere, existieren. Die Erfindung betrifft sowohl die Enantiomeren oder Diastereomeren als auch deren jeweiligen Mischungen. Die Racematformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Darüber hinaus ist die Bildung von atropen Isomeren möglich.

Bevorzugt sind Verbindungen der allgemeinen Formel (I),
in welcher

$R^1$ für eine Carboxylgruppe, oder
für eine $C_1$-$C_6$-Alkoxycarbonylgruppe steht,

$R^2$ für $C_1$-$C_8$-Alkyl (geradkettig oder verzweigt) steht, das gegebenenfalls durch Phenyl substituiert sein kann,

$R^3$ für Fluor, Chlor, Brom, Wasserstoff, $C_1$-$C_6$-Alkyl, Hydroxy, $C_1$-$C_4$-Alkoxy, Trifluormethyl oder Trifluormethoxy steht,

$R^4$ für Carboxyl oder Tetrazolyl steht

und

X für Sauerstoff steht,

und deren Salze.

Besonders bevorzugt sind Verbindungen der allgemeinen Formel (I),
in welcher

$R^1$ für eine Carboxylgruppe, oder für eine $C_1$-$C_4$-Alkoxycarbonylgruppe steht,

$R^2$ für $C_1$-$C_6$-Alkyl (geradkettig oder verzweigt) steht,

$R^3$ für Fluor, Chlor, Wasserstoff, Hydroxy, $C_1$-$C_4$-Alkyl, Methoxy, Trifluormethyl oder Trifluormethoxy steht,

$R^4$ für Tetrazolyl steht,

und

X für Sauerstoff steht,

und deren Salze.

Ganz besonders bevorzugt sind Verbindungen der allgemeinen Formel (I),
in welcher

$R^1$ für Carboxy, Methoxycarbonyl oder Ethoxycarbonyl steht,

$R^2$ für Ethyl oder Methyl steht,

$R^3$ für Fluor, Chlor, Methyl, Hydroxy, Trifluormethyl oder Trifluormethoxy steht,

$R^4$ für Tetrazolyl steht

und

X für Sauerstoff steht,

und deren Salze.

Die alkoxymethylsubstituierten Pyridonbiphenyle der allgemeinen Formel (I) werden hergestellt, indem man

[A] Pyridone der allgemeinen Formel (II)

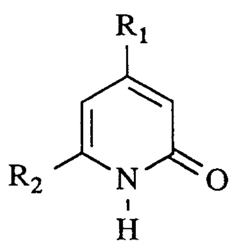

(II),

in welcher

$R^1$ und $R^2$ die oben angegebene Bedeutung haben,

mit Verbindungen der allgemeinen Formel (III)

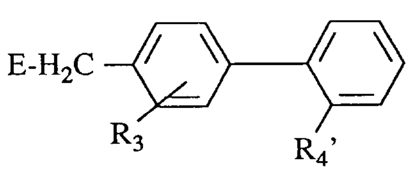

(III),

in welcher

$R^3$ die oben angegebene Bedeutung hat,
E für Chlor oder Brom steht,
und
$R^{4'}$ für $C_1$-$C_4$-Alkoxycarbonyl oder
für eine Gruppe der Formel

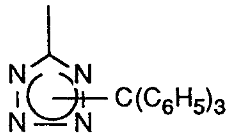

steht,
in inerten Lösemitteln, in Anwesenheit einer Base und gegebenenfalls unter Zusatz eines Katalysators umsetzt, oder
[B] im Fall, daß $R^4$ für Tetrazolyl steht
Verbindungen der allgemeinen Formel (IV)

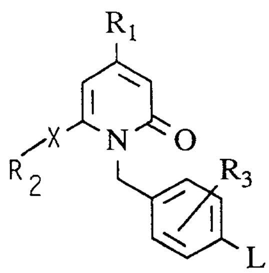

(IV),

in welcher
$R^1$, $R^2$ und $R^3$ die oben angegebene Bedeutung haben,
und
L für eine typische Abgangsgruppe, wie beispielsweise Brom, Iod, Methan-, Toluol-, Fluor- oder Trifluormethansulfonyloxy, vorzugsweise für Brom steht,
mit Verbindungen der allgemeinen Formel (V)

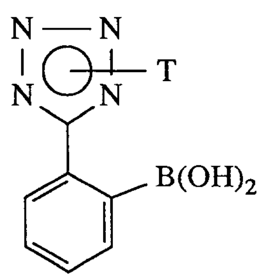

(V),

in welcher
T für Wasserstoff oder für die Triphenylmethylgruppe steht,
in inerten Lösemittel in Anwesenheit einer Base und metallkatalysiert umsetzt,
anschließend im Fall des freien Tetrazols ($R^4$/T)die Triphenylmethylgruppe mit Säuren in organischen Lösemitteln und/oder Wasser abspaltet,
im Fall der Carbonsäuren ($R^4$) den entsprechenden Ester verseift und die Verbindungen gegebenenfalls mit Basen in ihre Salze überführt.

Das erfindungsgemäße Verfahren kann durch folgendes Formelschema beispielhalt erläutert werden:

[A]

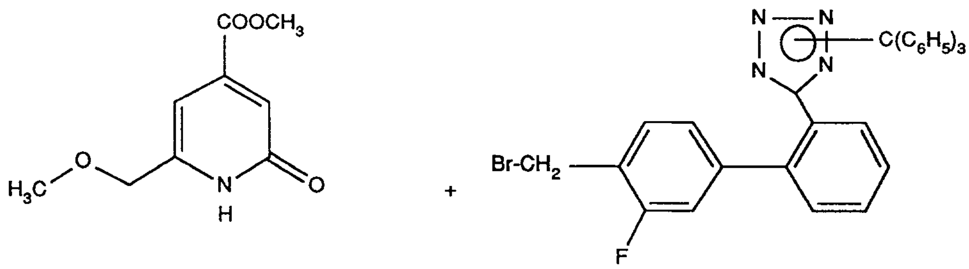

+

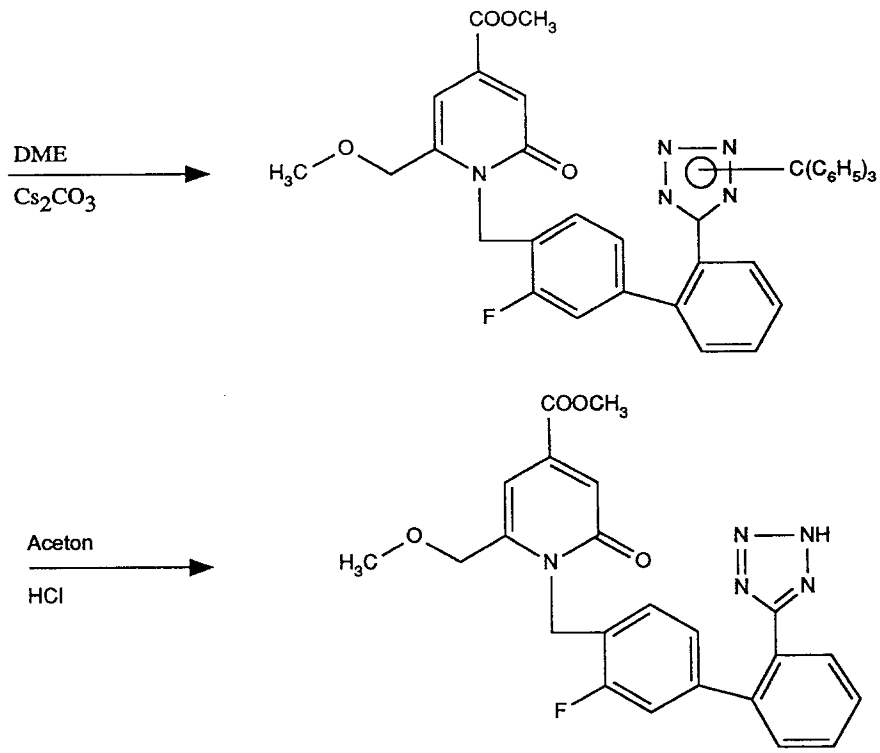
DME
$Cs_2CO_3$
Aceton
HCl

[B]

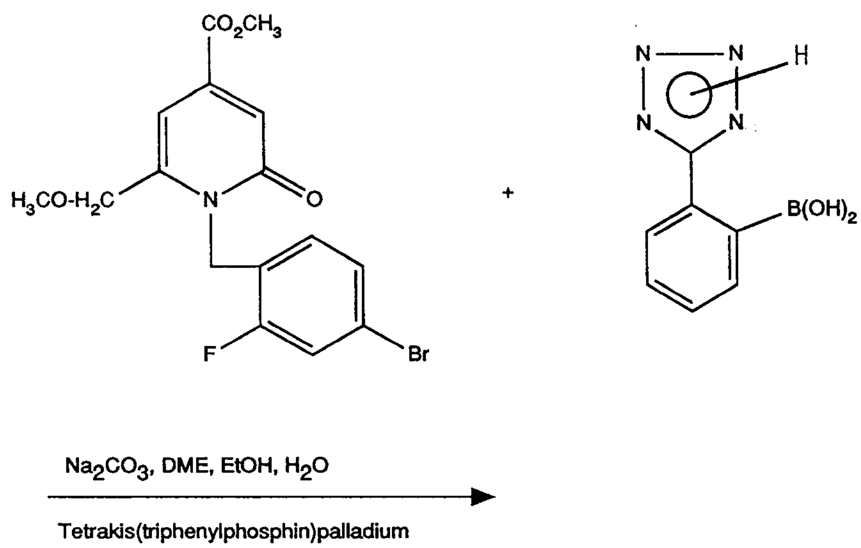
$Na_2CO_3$, DME, EtOH, $H_2O$
Tetrakis(triphenylphosphin)palladium

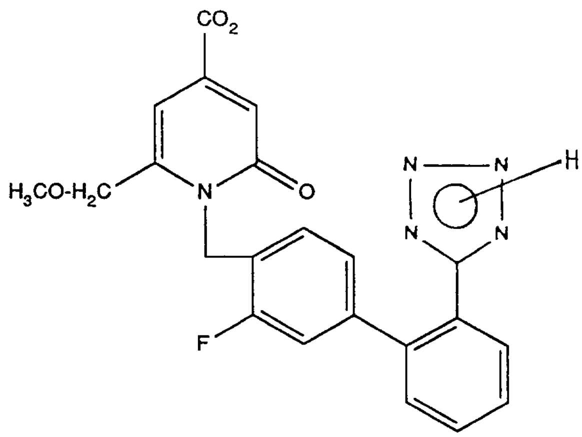

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Dimethoxyethan, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Tetrahydrofuran, Aceton, Dimethylformamid und Dimethoxyethan.

Als Basen für das erfindungsgemäße Verfahren [A] können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Alkali- oder Erdalkalicarbonate, Calcium- oder Caesiumcarbonat, oder Alkali- oder Erdalkalialkoholate oder -amide wie Natrium-oder Kaliummethanolat, Natrium- oder Kaliumethanolat oder Kalium-tert.butylat, oder Lithiumdiisopropylamid (LDA), oder organische Amine (Trialkyl($C_1$-$C_6$)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid einzusetzen. Bevorzugt sind Kaliumcarbonat, Natriumhydrid, Kalium-tert.-butylat oder Caesiumcarbonat.

Im allgemeinen setzt man die Base im Fall [A] in einer Menge von 0,05 mol bis 10 mol, bevorzugt von 1 mol bis 2 mol, bezogen auf 1 mol der Verbindung der Formel (III), ein.

Das erfindungsgemäße Verfahren [A] wird im allgemeinen in einem Temperaturbereich von -100 °C bis +100 °C, bevorzugt von 0 °C bis 80 °C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Als Losemittel für das erfindungsgemäße Verfahren [B] eignen sich übliche organische Lösermittel, die sich unter den Reaktionsbedingungen nicht verändern. Hierzu gehören bevorzugt Ether wie Diethylether, Dioxan, Tetrahydrofüran, Dimethoxyethan, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyrridin, Dimethylsulfoxid, Dimethylformamid, Hexamethylphosphorsäuretriamid, Acetonitril, Aceton oder Nitromethan. Ebenso ist es möglich, Gemische der genannten Losemittel zu verwenden.

Bevorzugt sind Tetrahydrofuran, Aceton, Dimethylformamid und Dimethoxyethan. Ebenso ist es möglich, in Gemischen der genannten Lösemittel mit Wasser zu arbeiten.

Das erfindungsgemäße Verfahren [B] wird im allgemeinen in einem Temperaturbereich von -20 °C bis +150 °C, bevorzugt von +40 °C bis +100 °C durchgeführt.

Als Katalysatoren eignen sich im allgemeinen Metallkomplexe des Nickels, Palladiums oder Platins, bevorzugt Palladium(O)-Komplexe wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Der Katalysator wird in einer Menge von 0,005 mol bis 0,2 mol, bevorzugt von 0,01 mol bis 0,05 mol, bezogen auf 1 mol der Verbindung der allgemeinen Formel (IV) eingesetzt.

Als Basen eignen sich im allgemeinen organische tert., nicht nucleophile Basen, wie beispielsweise Triethylamin oder Diisopropylethylamin oder anorganische Basen, wie Alkalicarbonate oder -hydroxide, beispielsweise Kalium-, Natrium- oder Thalliumcarbonat oder -hydroxid oder Alkoxide dieser Alkalimetalle. Bevorzugt sind Natriumcarbonat oder Kaliumcarbonat.

Im allgemeinen setzt man die Base in einer Menge von 1 mol bis 10 mol, bevorzugt von 1 mol bis 5 mol jeweils bezogen auf 1 mol der Verbindungen der Formel (IV) ein.

Gegebenenfalls werden die anorganischen Basen in wäßriger Lösung eingesetzt.

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen, oder in einer Lösung von Chlorwasserstoff in Dioxan.

Die Abspaltung erfolgt im allgemeinen in einem Temperaturbereich von 0°C bis 150°C, vorzugsweise von 20°C bis 100°C und Normaldruck.

Als Katalysatoren eignen sich Kalium- oder Natriumiodid, bevorzugt Natriumiodid.

Als Basen für die Verseifung der Ester eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofüran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Verbindungen der allgemeinen Formel (II) sind bekannt und können nach bekannten Methoden hergestellt werden.

Die Verbindungen der allgemeinen Formel (III) sind an sich bekannt oder können nach bekannten Methoden hergestellt werden.

Die Verbindung der Formel (V) ist im Fall (T = H) neu und kann hergestellt werden, indem man Phenyltetrazol zunächst unter Schutzgasatmosphäre in einem inerten Lösemittel und in Anwesenheit einer Base umsetzt und anschließend Borsäuretrimethylester zufügt und in einem letzten Schritt mit Säuren hydrolysiert.

Als Lösemittel eignen sich für das Verfahren aprotische Lösemittel wie Ether, beispielsweise Tetrahydrofüran, Diethylether, Toluol, Hexan oder Benzol. Bevorzugt ist Tetrahydrofuran.

Als Basen eignen sich prim-, sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, $C_1$-$C_4$-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt von -10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IV) sind größtenteils neu und können beispielsweise hergestellt werden, indem man Verbindungen der allgemeinen Formel (VI)

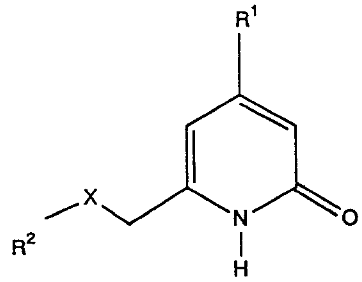

(VI)

in welcher

$R^1$, $R^2$ und X die oben angegebene Bedeutung haben,

mit Verbindungen der allgemeinen Formel (VII)

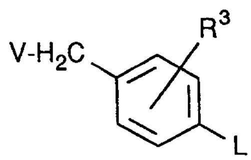

(VII)

in welcher

$R^3$ und L die oben angegebene Bedeutung haben

und

V für Halogen, vorzugsweise für Brom steht,

in inerten Lösemitteln, in Anwesenheit einer Base und/oder Katalysator umsetzt

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt für das Verfahren sind Tetrahydrofuran, Aceton, Dimethylformamid, Dimethoxyethan, Alkohole wie Methanol, Ethanol oder Propanol und/oder Wasser, Toluol und Methanol/Wasser.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium-oder Kaliummethanolat, Natrium- oder Kaliumethanolat oder Kalium-tert.butylat, Thalliumcarbonat- oder -hydroxid, oder Lithiumdiisopropylamid (LDA), oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind für das Verfahren Kaliumcarbonat, Natriumhydrid, Kalium-tert.-butylat oder Natriumcarbonat.

Im allgemeinen setzt man die Base in einer Menge von 0,05 mol bis 10 mol, bevorzugt von 1 mol bis 2 mol jeweils bezogen auf 1 mol der Verbindungen der Formel (VII) ein.

Das erfindungsgemäße Verfahren wird im allgemeinen in einem Temperaturbereich von -100 °C bis +100 °C, bevorzugt von 0 °C bis 80 °C unter Schutzgasatmosphäre durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Katalysatoren eignen sich für das Verfahren Kalium- oder Natriumiodid, bevorzugt Natriumiodid. Ebenso ist es möglich Phasen-Transfer-Katalysatoren wie beispielsweise Tetra-n-butylammoniumbromid oder Kronenether einzusetzen.

Der Katalysator wird in einer Menge von 0,1 mol bis 10 mol, bevorzugt von 1 mol bis 2 mol, bezogen auf 1 mol der Verbindung der allgemeinen Formel (VII) eingesetzt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen alkoxymethylsubstituierten Pyridonbiphenyle zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüber hinaus inhibieren sie die Proliferation von glatten Muskelzellen.

Sie können deshalb in Arzneimitteln zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37 °C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewerte. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Innubationszeit in die Bäder appliziert wird. Jeder Aorterring wird dabei ganztägig mit immer dem gleichen Agonisten stimuliert.

Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

Zur Bestimmung der $IC_{50}$-Werte wird die Wirkstoffkonzentration errechnet, die bei sequentieller Verdünnung des Wirkstoffes halbmaximale Hemmung der durch 10 % FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Losungen, unter Verwendung inerter, nicht-toxischer, pharmazeutisch geeigneter Trägerstoffe oder Losemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fallen ausreichend sein, mit weniger als der vorgenannten Mindestmenge auszukommen, wahrend in anderen Fallen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

N-(1-Hydroxy-2-methyl-prop-2-yl)-2-methoxy-benzoesäureamid

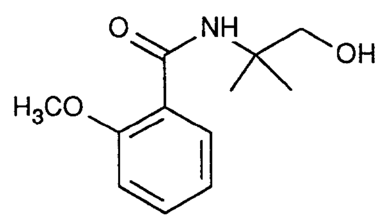

15,2 g (100 mmol) 2-Methoxy-benzoesäure werden in 300 ml Dichlormethan gelöst und bei 0 °C mit 14,2 g (105 mmol) 1-Hydroxy-benzoesäuretriazol x 1 $H_2O$ und 21,66 g (105 mmol) N,N-Dicyclohexylcarbodiimid verrührt. Die so erhaltene Suspension wird 0,5 h bei Raumtemperatur gerührt, wieder auf 0 °C gekühlt und mit einer Losung von 9,89 g (111 mmol) 1-Hydroxy-2-methyl-2-propylamin und 12,65 g (125 mmol) Triethylamin in 300 ml Dichlormethan versetzt. Nach 1 h ist die Reaktion vollständig. Die Reaktionsmischung wird mit 1 M Salzsäure und gesättigter Natriumhydrogencarbonatlösung gewaschen, über Natriumsulfat getrocknet und im Vakuum eingeengt. Das Rohprodukt wird mit Petrolether verrührt, abgesaugt, mit dem Losemittel nachgewaschen und im Hochvakuum getrocknet.

Beispiel II

4,5-Dihydro-5,5-dimethyl-2-(2-methoxyphenyl)-oxazol

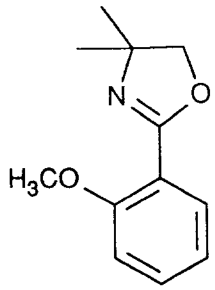

16,0 g (71,7 mmol) der Verbindung aus Beispiel I wird bei Raumtemperatur mit 17,1 ml (283,4 mmol) Thionylchlorid versetzt und 3 h gerührt. Darauf wird überschüssiges Reagenz abgedampft, der Rückstand mit 500 ml Ether ausgerührt und abgesaugt. Der Feststoff wird in Wasser gelöst, mit Ether überschichtet und die korrespondierende Base mit 2 M Natronlauge freigesetzt. Nach dreimaliger Extraktion der wäßrigen Phase mit Essigester werden die vereinigten organischen Phasen mit Natriumsulfat getrocknet, eingedampft und im Hochvakuum vom Restlösemittel befreit.

Beispiel III

4,5-Dihydro-5,5-dimethyl-2-(3'-fluor-4'-methyl-biphenyl-2-yl)oxazol

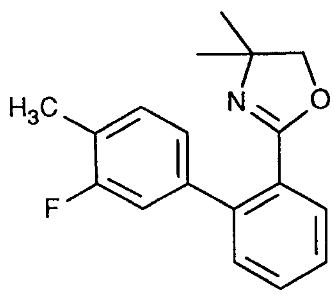

14,7 g (605,7 mol) Magnesiumspäne werden unter Argon in 50 ml Tetrahydrofuran p.a. vorgelegt und unter Rühren mit 117,7 g (623 mmol) 4-Brom-2-fluor-toluolin 500 ml Tetrahydrofuran p.a. versetzt. Bei 35 - 40 °C entsteht innerhalb von 2 h eine klare Lösung. Dazu tropft man bei Raumtemperatur eine Losung von 74,0 g (360,5 mmol) der Verbindung aus Beispiel II in 500 ml Tetrahydrofuran p.a und rührt anfangs unter gelinder Kühlung bei ca. 25 °C 16 h nach. Das Lösemittel wird abgedampft und das Rohprodukt bei 10 °C in 600 ml Essigester und 800 ml gesättigter Ammoniumchloridlösung nachgewaschen, mit Natriumsulfat getrocknet und im Vakuum eingedampft. Zur Reinigung nimmt man in 600 ml Ether auf, saugt etwaigen festen Rückstand ab und zieht das Rohprodukt durch mehrfache Extraktion mit 2 M Salzsäure in die wäßrige Phase. Diese wird mit Ether überschichtet und mit Natronlauge auf pH 13 gestellt. Nach dreimaliger Extraktion mit Ether wird mit Natriumsulfat getrocknet, eingedampft und Restlösemittel im Hochvakuuum entfernt.

Beispiel IV

2-(3-Fluor-4-methylphenyl)-benzonitril

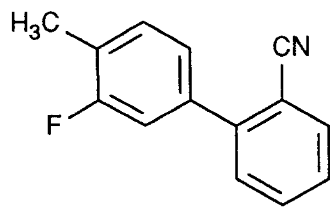

97,0 g (343 mmol) der Verbindung aus Beispiel III werden in 500 ml Pyridin vorgelegt und bei 0 ° C unter Rühren mit 31,3 ml (343 mmol) Phosphoroxychlorid versetzt. Das Gemisch wird langsam erwärmt und schließlich 1 h unter Rückfluß gekocht. Nach Abkühlung auf Raumtemperatur setzt man Ether und so viel 1 M Salzsäure zu, daß der pH der wäßrigen Phase bei 1,5 liegt. Die organische Phase wird noch dreimal mit 1 M Schwefelsäure gewaschen, mit Natriumsulfat getrocknet, einrotiert und im Hochvakuum vom Restlösemittel befreit.

Beispiel V

5-(3'-Fluor-4'-methyl-biphenyl-2-yl)-1H-tetrazol

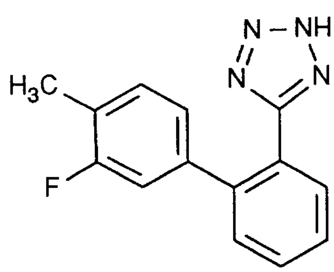

2,26 g (10,7 mmol) der Verbindung aus Beispiel IV werden mit 3,48 g (53,6 mmol) Natriumazid und 7,37 g (53,6 mmol) Triethylammoniumchlorid in 30 ml Dimethylformamid p.a. 24 h unter Rückfluß gekocht Nach Abkühlung verteilt man zwischen Ether und 1 M Schwefelsäure, wäscht die organische Phase mit Wasser nach, trocknet über Natriumsulfat und dampft das Lösemittel ab. Das Rohprodukt wird in Toluol ausgerührt und nach dem Absaugen im Vakuum getrocknet (1,89 g, 7,2 mmol). Die Mutterlauge wird einrotiert und erneut wie oben gereinigt (0,43 g, 1,7 mmol).

Beispiel VI

5-(3-Fluor-4-methyl-biphenyl-2-yl)-2-triphenylmethyl-1H-tetrazol

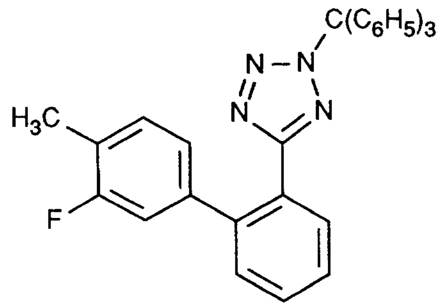

50,55 g (199,2 mmol) der Verbindung aus Beispiel V werden mit 58,58 g (210,0 mmol) Triphenylchlormethan und 33,2 ml (239,0 mmol) Triethylamin in 700 ml Dichlormethan 17 h bei Raumtemperatur gerührt. Die Reaktionsmischung wird einmal mit Wasser und einmal mit 1 M wäßriger Zitronensäure gewaschen, mit Natriumsulfat getrocknet, einrotiert und im Hochvakuum vom Restlösemittel befreit

Beispiel VII

5-(4'-Brommethyl-3'-fluor-biphenyl-2-yl)-2-triphenylmethyl-1H-tetrazol

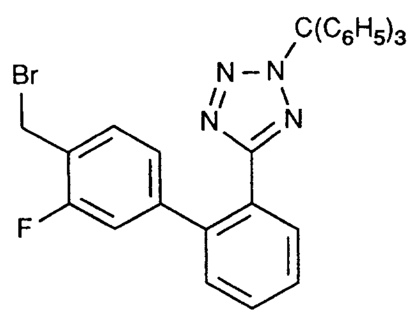

82,90 g (173,2 mmol) der Verbindung aus Beispiel VI werden mit 30,84 g (173,2 mmol) N-Bromsuccinimid und 0,87 g (5,3 mmol) Azobisisobutyronitril als Radikalstarter 6 h in 1 l Tetrachlormethan unter Rückfluß gekocht. Nach dem Abkühlen wird das ausgefallene Succinimid abgesaugt und mit Tetrachlormethan gewaschen. Das Filtrat wird eingedampft und im Hochvakuum getrocknet.

Beispiel VIII

2,4-Dioxo-5-methoxy-pentansäuremethylester

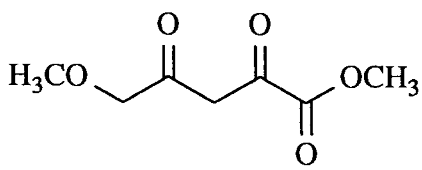

Zu einer Lösung von 59,4 g (1,1 mol) Natriummethylat in 200 ml Methanol tropft man unter Rückfluß eine Lösung von 88,1 g (1 mol) Methoxyaceton und 118,1 g (1 mol) Oxalsäuredimethylester in 150 ml Methanol innerhalb 30 min zu und erhitzt weitere 2 h unter Rückfluß. Das erkaltete Reaktionsgemisch wird auf 800 ml Eiswasser gegeben, mit konz. Schwefelsäure auf pH 1,5 eingestellt und dreimal mit je 500 ml Essigester gewaschen. Die vereinigten organischen Phasen werden mit 500 ml 1%iger Natriumhydrogencarbonatlösung gewaschen, mit Natriumsulfat getrocknet und destilliert.
(Sdp.125 ° C/ 20 mbar).

Beispiel IX

3-Cyano-4-methoxycarbonyl-6-methoxymethyl-2-oxo-1,2-dihydropyridin

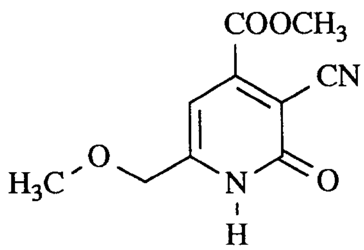

18,9 g (0,224 mol) Cyanoacetamid, 31 g (0,224 mol) Kaliumcarbonat und 34,6 g (0,224 mol) der Verbindung aus Beispiel VIII werden in 200 ml Aceton 2 h unter Rückfluß erhitzt Das Reaktionsgemisch wird in 500 ml Wasser gelöst, 2 mal mit je 250 ml Diethylether gewaschen, die wäßrige Produktphase mit konz. Salzsäure auf pH = 1,5 eingestellt und der Niederschlag abgesaugt.
Schmp.: 200-203 ° C (Zers.)

Beispiel X

4-Carboxy-6-methoxymethyl-2-oxo-1,2-dihydro-pyridin

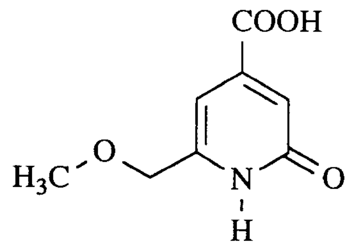

10,41 g (46,8 mmol) der Verbindung aus Beispiel IX werden in 20 ml Wasser, 16,7 ml konz. Schwefelsäure 4 h unter Rückfluß erhitzt. Das Reaktionsgemisch wird auf 700 ml Eiswasser gegossen, mit 5 N Natronlauge auf pH = 2 gestellt, die Lösung mit Natriumchlorid gesättigt,ein schleimiger Niederschlag abgesaugt und gefriergetrocknet.

Beispiel XI

4-Methoxycarbonyl-6-methoxymethyl-2-oxo-1,2-dihydropyridin

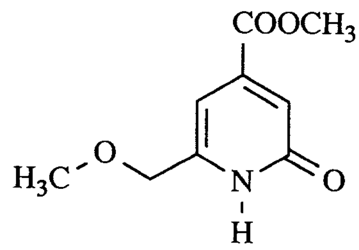

Zu einer Suspension von 8,95 g (48,9 mmol) der Verbindung aus Beispiel X in 100 ml Methanol tropft man 4 ml (55,4 mmol) Thionylchlorid und rührt 18 h bei 50 °C. Das Reaktionsgemisch wird zur Trockene eingeengt und an 100 g Kieselgel 60 mit Dichlormethan / Methanolgemischen (20:1 bis 7:1) chromatographiert.
Schmp.: 165 °C

Beispiel XII

4-Methoxycarbonyl-6-methoxymethyl-2-oxo-1-{[3-fluor-2'-(N-triphenylmethyltetrazol-5-yl)-biphenyl-4-yl]-methyl}-1,2-dihydropyridin

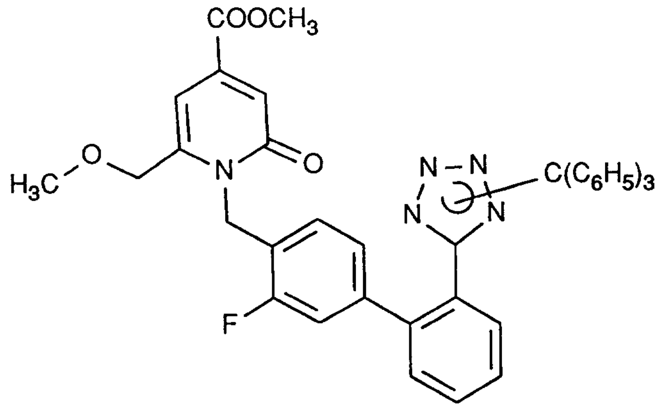

Eine Suspension von 1,99 g (10,1 mmol) der Verbindung aus Beispiel XI und 3,29 g (10,1 mmol) Caesiumcarbonat in 20 ml Dimethoxyethan wird 10 min bei Raumtemperatur gerührt, mit einer Losung von 5,84 g (10,1 mmol) der Verbindung aus Beispiel VII in 20 ml Dimethoxyethan versetzt, 20 h bei Raumtemperatur gerührt, dann 2 h unter Rückfluß erhitzt. Nach Zugabe von 100 ml Wasser wird die Reaktionslösung mit 3 x 100 ml Essigester extrahiert. Trocknen, Einengen und Kieselgelchromatographie (Petrolether : Essigester = 5:1 → 1:1) der organischen Phasen liefern einen farblosen Schaum.

Beispiel XIII

2-(Tetrazol-5-yl)phenylboronsäure

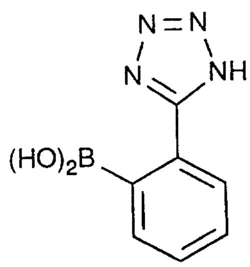

Eine Lösung von 2,9 g (20 mmol) 5-Phenyltetrazol in 50 ml THF wird bei - 5 °C unter Argon mit 17,6 ml (44 mmol) einer 2,5 M Lösung von n-Butyllithium in n-Hexan versetzt. Man läßt 30 min bei -5 °C bis 0 °C rühren und gibt bei dieser Temperatur 10 ml (88 mmol) Borsäuretrimethylester hinzu. Dann wird das Kühlbad entfernt, und die Lösung bei Raumtemperatur mit 10 ml halbkonzentrierter Salzsäure versetzt Nach 1 h wird mit 100 ml Essigester extrahiert, die organische Phase abgetrennt und die wäßrige Phase zweimal mit je 20 ml Essigester extrahiert. Die vereinigten organischen Phasen werden über Natriumsulfat getrocknet, eingeengt und der Rückstand an Kieselgel mit Toluol / Eisessig / Methanol (38 : 0,1 : 2) gereinigt.

Ausbeute: 2,65 g (70% d.Th.)

$R_f$ = 0,26 (Toluol/Methanol/Eisessig = 32:8:1)

$^{13}C$-NMR: $\delta$ = 156,7; 137,9; 133,5; 129,8; 128,9; 127,7; 126,9 ppm.

Beispiel XIV

4-Methoxycarbonyl-6-methoxymethyl-2-oxo-1-(2-fluor-4-iod-phenylmethyl)-1,2-dihydropyridin

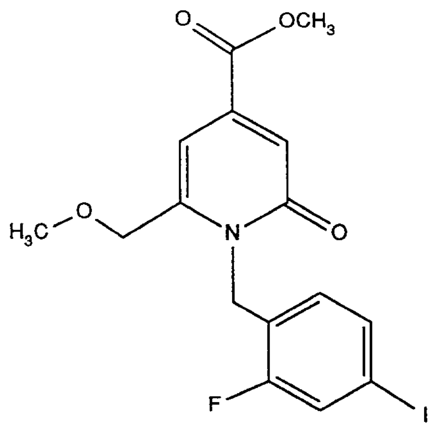

Eine Lösung von 2 g (10,14 mmol) der Verbindung aus Beispiel XI, 4,12 g (13,1 mmol) 2-Fluor-4-iodbenzylbromid und 4,89 g (15 mmol) Cäsiumcarbonat in 20 ml THF wird unter Argon 16 h bei 20 °C gerührt. Anschließend wird das Solvens im Vakuum entfernt, der Rückstand mit Dichlormethan/Wasser aufgenommen, die wäßrige Phase einmal mit Dichlormethan extrahiert, die vereinigten organischen Phasen über Natriumsulfat getrocknet und eingeengt. Der Rückstand wird an Kieselgel mit Petrolether/Essigester (5:1 und 3:1) gereinigt

Ausbeute: 1,3 g (30 % der Theorie)

$R_f$: 0,16 (Petrolether/Essigester = 3:1)

Beispiel XV

4-Methoxycarbonyl-6-methoxymethyl-2-oxo-1-(2-chlor-4-iod-phenylmethyl)-1,2-dihydropyridin

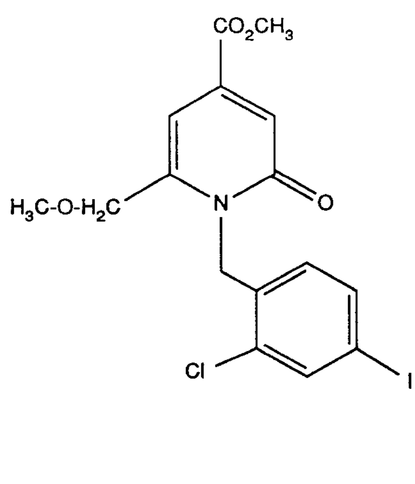

In Analogie zur Vorschrift des Beispiels XIV wird die Titelverbindung hergestellt.

$R_f$: 0,25 (Lösungsmittel:Petrolether:Essigester 1:2)

Beispiel XVI

3-Chlor-4-trifluormethylsulfonyloxy-benzoesäure-methylester

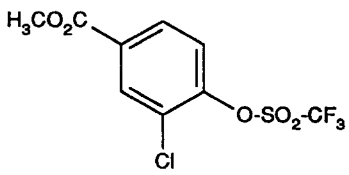

Zu einer Lösung von 5,49 g 3-Chlor-4-hydroxy-benzoesäure-methylester (29,4 mmol) in 15 ml Pyridin werden bei 0 ° C langsam 5,5 ml Trifluormethansulfonsäureanhydrid (33 mmol) getropft. Nach 5 min Rühren bei 0 ° C und 4 h bei RT wird das Reaktionsgemisch zwischen Wasser und Ether verteilt. Die organische Phase wird nacheinander mit Wasser, 10 % Salzsäure, Wasser und gesättigter Kochsalzlösung gewaschen, über Natriumsulfat getrocknet, eingeengt und mit Methylenchlorid über Kieselgel chromatographiert, um 8,93 g eines hellgelben dünnflüssigen Öls zu erhalten [95,2 % d. Th., $R_f$ 0,63 (Hexan:Essigester = 3:1)].

Beispiel XVII

5-(2'-Chlor-4'-methoxycarbonyl-biphenyl-2-yl)-2-triphenylmethyl-1H-tetrazol

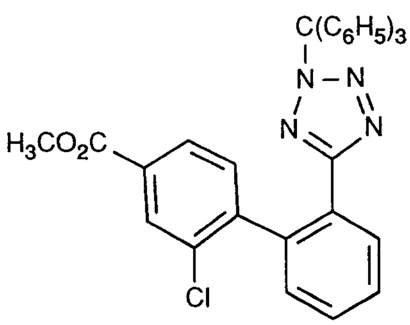

Durch eine Lösung von 1,00 g (3,14 mmol) der Verbindung aus Beispiel XVI in 50 ml Toluol wird Argon geleitet Nach Zugabe von 168 mg Pd $(C_6H_5)_3)_4$ (0,146 mmol), 6 ml Methanol, 1,63 g (3,77 mmol) 2-(N-Triphenylmethyl-tetrazol-5-yl)-phenyl-boronsäure und einer Lösung von 333 mg (3,14 mmol) Natriumcarbonat in 4 ml entgastem Wasser wird die Emulsion über Nacht bei 100 ° C gerührt. Zugabe der gleichen Menge Katalysator, gefolgt von 2,5 h Rühren bei 100 ° C vervollständigt die Reaktion. Das Reaktionsgemisch wird zwischen Wasser und Essigester verteilt, die organische Phase wird mit verd. Natriumcarbonat-Lösung und gesättigter Kochsalzlösung gewaschen, über Natriumsulfat getrocknet, eingeengt und über Kieselgel chromatographiert (Hexan:Essigester = 10:1 bis 8:1), um 10,1 g eines hellgelben Feststoffs zu erhalten [57,9 % d.Th; $R_f$ 0,46 (Hexan:Essigester = 3:1)].

Beispiel XVIII

5-(2'-Chlor-4'-hydroxymethyl-biphenyl-2-yl)-2-triphenylmethyl-1H-tetrazol

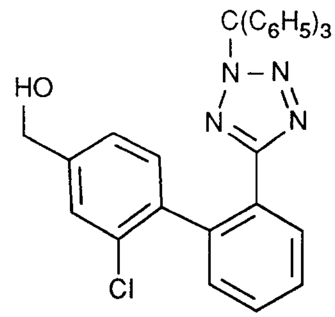

Eine Lösung von 22,0 g (39,6 mmol) der Verbindung aus Beispiel XVII in 180 ml THF wird mit 1,27 g Methanol (39,6 mmol) und 1,29 g Lithiumborhydrid (59,4 mmol) versetzt, dann 30 min bei RT und 1 h unter Rückfluß gerührt. Zugabe weiterer 0,63 g Methanol (20 mmol) und 1 h Rühren unter Rückfluß vervollständigt die Reaktion. Das Reaktionsgemisch wird eingeengt; der Rückstand wird in 200 ml Methylenchlorid aufgenommen und unter einem kräftigen Argon-Strom mit Eisbad langsam mit 100 ml in Kaliumhydrogensulfat-Lösung versetzt. Nach Trennen der Phasen wird die wäßrige Phase mit Methylenchlorid extrahiert. Die vereinten organischen Phasen werden mit gesättigter Kochsalzlösung gewaschen, über Natriumsulfat getrocknet und eingeengt, um 20,5 g weiße Kristalle zu ergeben [98,19 % d.Th.; Fp. 186-7 °C (Zers.); $R_f$ 0,15 (Hexan:Essigester = 3:1)].

Beispiel XIX

5-(4'-Brommethyl-2'-chlor-biphenyl-2-yl)-2-triphenylmethyl-1H-tetrazol

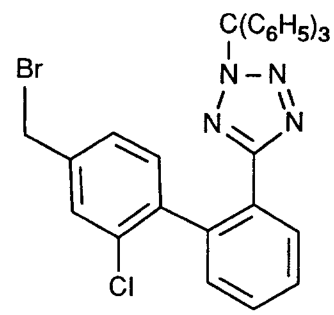

Zu einer Lösung von 11,2 g Triphenylphosphin (42,5 mmol) in 100 ml Methylenchlorid werden im Eisbad unter Argon erst 6,79 g Brom (42,5 mmol), dann 20,4 g der Verbindung aus Beispiel XVII in 300 ml Methylenchlorid getropft. Nach 1 h Rühren bei Raumtemperatur wird das Reaktionsgemisch durch Kieselgel filtriert und mit Methylenchlorid eluiert Einengen des Filtrats und Digerieren des Rückstands mit Hexan ergeben 15,8 g weiße Kristalle [68,9 % d.Th.; Fp. 15-60 °C; $R_f$ 0,40 (Hexar/Essigester = 3:1)].

Beispiel XX

4-Methoxycarbonyl-6-methoxymethyl-2-oxo-1-{[2-chlor-2'-(N-triphenylmethyltetrazol-5-yl)biphenyl-4-yl]-methyl}-1,2-dihydropyridin

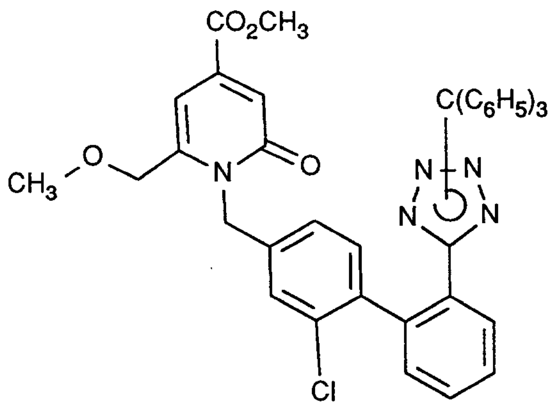

In Analogie zur Vorschrift des Beispiels XII werden aus 0,46 g (2,33 mmol) der Verbindung aus Beispiel XI und 1,38 g (2,33 mmol) der Verbindung aus Beispiel XIX 0,17 g der Titelverbindung erhalten [10 % der Theorie; $R_f$ 0,31 (Hexan:Essigester = 1:1)].

Herstellungsbeispiele

Beispiel 1

4-Methoxycarbonyl-6-methoxymethyl-2-oxo-1-[(3-fluor-2'-tetrazol-5-yl-biphenyl-4-yl)methyl]-1,2-dihydropyridin

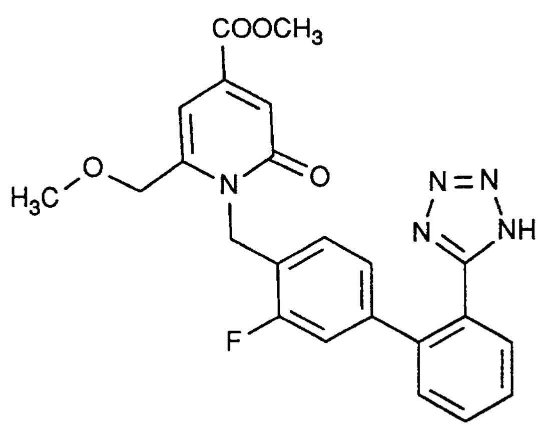

3,19 g (4,61 mmol) der Verbindung aus Beispiel XII werden in 30 ml Methanol und 1,53 ml (18,4 mmol) 12 N Salzsäure gelöst Nach 1 h wird die Suspension gekühlt und der Niederschlag abgesaugt.

Beispiel 2

4-Carboxy-6-methoxymethyl-2-oxo-1-[(3-fluor-2'-tetrazol-5-yl-biphenyl-4-yl)methyl]-1,2-dihydropyridin- Dinatriumsalz

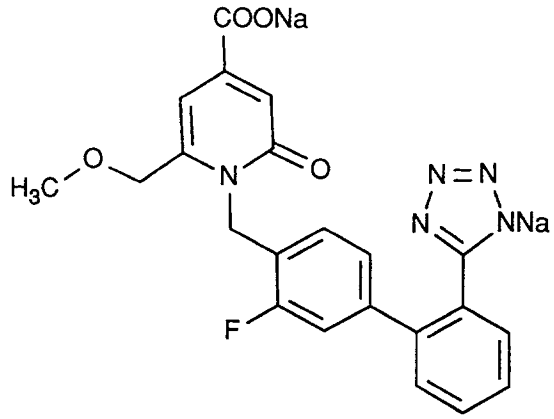

1,04 g (2,4 mmol) der Verbindung aus Beispiel 1 werden in 5 ml Methanol, 5 ml Tetrahydrofuran, 4,8 ml 1 N Natronlauge 2 h bei Raumtemperatur gerührt. Das Lösemittel wird abdestilliert und der wäßrige Rückstand gefriergetrocknet

Beispiel 3

4-Carboxy-6-methoxymethyl-2-oxo-1-[(3-fluor-2'-tetrazol-5-yl-biphenyl-4-yl)methyl]-1,2-dihydropyridin

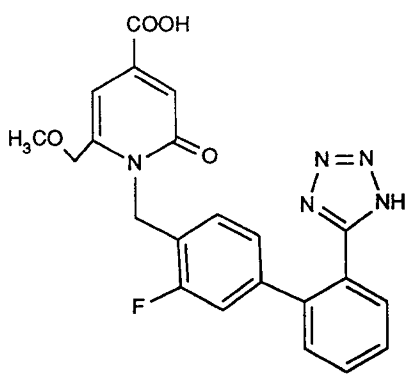

Eine Lösung von 1,2 g (2,78 mmol) der Verbindung aus Beispiel XIV in 20 ml DME wird sukzessive mit 321 mg Tetrakistriphenylphosphinpalladium(0), 8,34 ml (16,7 mmol) 2 M Natriumcarbonatlösung, 634 mg (3,34 mmol) der Verbindung des Beispiels XIII und 1,5 ml Ethanol versetzt und 16 h unter Rückfluß erhitzt. Nach Abkühlen wird die Reaktionsmischung über Kieselgur abgesaugt, mit Methanol nachgewaschen, das Solvens entfernt und an Kieselgel mit Toluol/Essigester/Eisessig (30:10:1 und 20:20:1) gereinigt.

Ausbeute: 285 mg (24 % der Theorie)

$R_f$: 0,11 (Toluol/Essigester/Eisessig = 10:30:1)

Die in der folgenden Tabelle aufgefürten Verbindungen wurden analog zu den Beispielen 1,2 und 3 hergestellt:

Tabelle:

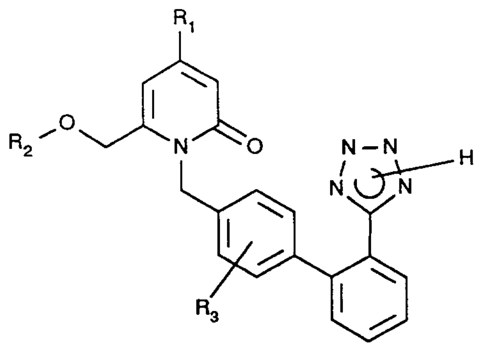

| Bsp.-Nr. | $R^1$ | $R^2$ | $R^3$ | Salz /Säure |
|---|---|---|---|---|
| 4 | $CO_2H$ | $CH_3$ | 2-Cl | Säure |
| 5 | $CO_2H$ | $CH_3$ | 3-Cl | Säure |
| 6 | $CO_2H$ | $CH_3$ | 2-Me | Di-Na-Salz |
| 7 | $CO_2CH_3$ | $CH_3$ | 3-Me | Säure |
| 8 | $CO_2H$ | $C_2H_5$ | 2-OH | Säure |
| 9 | $CO_2H$ | $C_2H_5$ | 3-OH | Säure |
| 10 | $CO_2CH_3$ | $C_2H_5$ | 2-$CF_3$ | Mono-K-Salz |
| 11 | $CO_2H$ | $C_2H_5$ | 3-$CF_3$ | Di-Li-Salz |
| 12 | $CO_2C_2H_5$ | $C_2H_5$ | 2-$OCF_3$ | Säure |
| 13 | $CO_2H$ | $C_2H_5$ | 3-$OCF_3$ | Säure |

Fortsetzung Tabelle:

| Bsp.-Nr. | $R^1$ | $R^2$ | $R^3$ | Salz /Säure |
|---|---|---|---|---|
| 14 | $CO_2H$ | $CH_3$ | 2-F | Säure |
| 15 | $CO_2CH_3$ | $CH_3$ | 2-F | Säure |
| 16 | $CO_2CH_3$ | $CH_3$ | 2-F | mono K-Salz |
| 17 | $CO_2CH_3$ | $CH_3$ | 2-Cl | Säure |
| 18 | $CO_2CH_3$ | $CH_3$ | 2-Cl | mono K-Salz |
| 19 | $CO_2H$ | $CH_3$ | 3-Cl | di K-Salz |
| 20 | COOH | $CH_3$ | 3-F | mono K-Salz |
| 21 | $CO_2CH_3$ | $CH_3$ | 3-Cl | Säure |
| 22 | $CO_2CH_3$ | $CH_3$ | 3-Cl | mono K-Salz |

## Patentansprüche

1. Alkoxymethylsubstituierte Pyridonbiphenyle der allgemeinen Formel

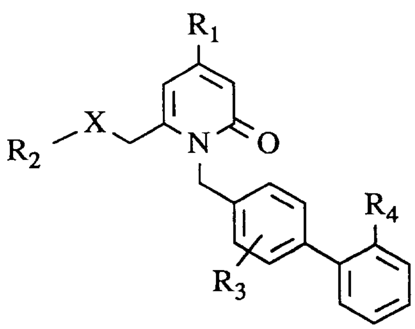

(I),

in welcher

$R^1$ für eine Carboxylgruppe steht, oder für eine $C_1$-$C_8$-Alkoxycarbonylgruppe steht,

$R^2$ für $C_1$-$C_{10}$-Alkyl (geradkettig oder verzweigt) steht, das gegebenenfalls durch Phenyl substituiert ist,

$R^3$ für Halogen, Wasserstoff, $C_1$-$C_6$-Alkyl, Hydroxy, $C_1$-$C_8$-Alkoxy, Trifluormethyl oder Trifluormethoxy steht,

$R^4$ für Carboxyl oder für Tetrazolyl steht

und

X für Sauerstoff oder Schwefel steht,

und deren Salze.

**2.** Alkoxymethylsubstituierte Pyridonbiphenyle nach Anspruch 1, in welcher
$R^1$ für eine Carboxylgruppe, oder für eine $C_1$-$C_6$-Alkoxycarbonylgruppe steht,
$R^2$ für $C_1$-$C_8$-Alkyl (geradkettig oder verzweigt) steht, das gegebenenfalls durch Phenyl substituiert sein kann,
$R^3$ für Fluor, Chlor, Brom, Wasserstoff, $C_1$-$C_6$-Alkyl, Hydroxy, $C_1$-$C_4$-Alkoxy, Trifluormethyl oder Trifluormethoxy steht,
$R^4$ für Carboxyl oder Tetrazolyl steht
und
X für Sauerstoff steht,
und deren Salze.

**3.** Alkoxymethylsubstituierte Pyridonbiphenyle nach Anspruch 1, in welcher
$R^1$ für eine Carboxylgruppe, oder für eine $C_1$-$C_4$-Alkoxycarbonylgruppe steht,
$R^2$ für $C_1$-$C_6$-Alkyl (geradkettig oder verzweigt) steht,
$R^3$ für Fluor, Chlor, Wasserstoff, Hydroxy, $C_1$-$C_4$-Alkyl, Methoxy, Trifluormethyl oder Trifluormethoxy steht,
$R^4$ für Tetrazolyl steht
und
X für Sauerstoff steht,
und deren Salze.

**4.** Alkoxymethylsubstituierte Pyridonbiphenyle nach Anspruch 1, in welcher
$R^1$ für Carboxy, Methoxycarbonyl oder Ethoxycarbonyl steht,
$R^2$ für Ethyl oder Methyl steht,
$R^3$ für Fluor, Chlor, Methyl, Hydroxy, Trifluormethyl oder Trifluormethoxy steht,
$R^4$ für Tetrazolyl steht
und
X für Sauerstoff steht,
und deren Salze.

**5.** Alkoxymethylsubstituierte Pyridonbiphenyle nach Anspruch 1 zur therapeutischen Anwendung.

**6.** Verfahren zur Herstellung von alkoxymethylsubstituierten Pyridonbiphenylen nach Anspruch 1 dadurch gekennzeichnet, daß man

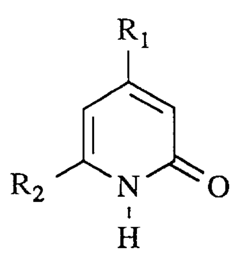

(II),

in welcher
$R^1$ und $R^2$ die oben angegebene Bedeutung haben,
mit Verbindungen der allgemeinen Formel (III)

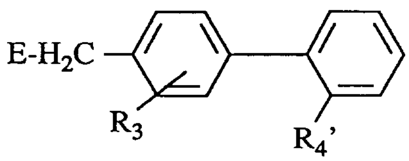

(III),

in welcher

$R^3$ die oben angegebene Bedeutung hat,

E für Chlor oder Brom steht

und

$R^{4'}$ für $C_1$-$C_4$-Alkoxycarbonyl oder für eine Gruppe der Formel

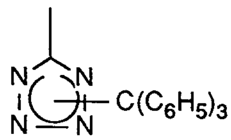

steht,

in inerten Lösemitteln, in Anwesenheit einer Base und gegebenenfalls unter Zusatz eines Katalysators umsetzt, oder

[B] im Fall, daß $R^4$ für Tetrazolyl steht

Verbindungen der allgemeinen Formel (IV)

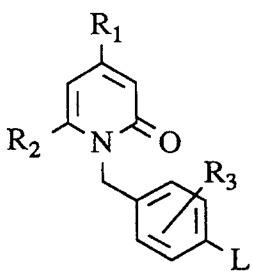

(IV),

in welcher

$R^1$, $R^2$ und $R^3$ die oben angegebene Bedeutung haben,

und

L für eine typische Abgangsgruppe, wie beispielsweise Brom, Iod, Methan-, Toluol-, Fluor- oder Trifluormethansulfonyloxy, vorzugsweise für Brom steht,

mit Verbindungen der allgemeinen Formel (V)

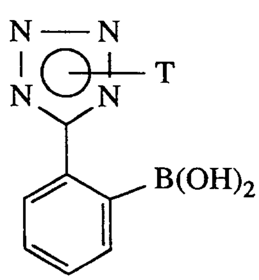

(V),

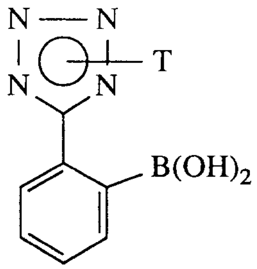

(V),

in welcher

T für Wasserstoff oder für die Triphenylmethylgruppe steht,

in inerten Lösemittel in Anwesenheit einer Base und metallkatalysiert umsetzt,

anschließend im Fall des freien Tetrazols ($R^4$/T)die Triphenylmethylgruppe mit Säuren in organischen Lösemitteln und/oder Wasser abspaltet,

im Fall der Carbonsäuren ($R^4$/$R^1$) den entsprechenden Ester verseift und die Verbindungen gegebenenfalls mit Basen in ihre Salze überführt

**7.** Arzneimittel enthaltend mindestens ein alkoxymethylsubstituiertes Pyridonbiphenyl nach Anspruch 1.

**8.** Arzneimittel nach Anspruch 7 zur Behandlung von Bluthochdruck sowie Atherosklerose.

**9.** Verwendung von alkoxymethylsubstituierten Pyridonbiphenylen nach Anspruch 1 zur Herstellung von Arzneimitteln.

**10.** Verwendung nach Anspruch 9 zur Herstellung von antihypertensiven und anti-atherosklerotischen Mitteln.

Europäisches Patentamt

# EUROPÄISCHER RECHERCHENBERICHT

Nummer der Anmeldung

EP 93 11 6416

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 542 059 (BAYER AG) 19. Mai 1993 * Beispiele XCIX, CXXIV, CXLVIII, CLXXVI, CXCV * * Ansprüche 1-11 * --- | 1-10 | C07D401/10 A61K31/44 |
| D,Y | EP-A-0 500 297 (FISONS PLC) 26. August 1992 * Beispiele 38, 51 * * Ansprüche 1,2 * --- | 1-10 | |
| D,Y | EP-A-0 487 745 (MEIJI SEIKA KABUSHIKI KAISHA) 3. Juni 1992 * Anspruch 1 * --- | 1-10 | |
| A | EP-A-0 445 811 (TAKEDA CHEMICAL INDUSTRIES, LTD.) 11. September 1991 * Anspruch 1 * ----- | 1-10 | |

| RECHERCHIERTE SACHGEBIETE (Int. Cl.5 ) |
|---|
| C07D A61K |

Der vorliegende Recherchenbericht wurde für alle Patentansprüche erstellt

| Recherchenort | Abschlußdatum der Recherche | Prüfer |
|---|---|---|
| MUENCHEN | 30 NOVEMBER 1993 | HARTRAMPF G.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)